(19) Europäisches Patentamt
European Patent Office
Office européen des brevets

(11) Publication number: 0 260 572 A1

(12) # EUROPEAN PATENT APPLICATION

(21) Application number: 87113080.3

(51) Int. Cl.[4]: C07C 69/14 , C07C 67/08

(22) Date of filing: 08.09.87

(30) Priority: 17.09.86 US 908157

(43) Date of publication of application:
23.03.88 Bulletin 88/12

(84) Designated Contracting States:
BE DE FR GB IT NL

(71) Applicant: BASF Corporation
9 Campus Drive
Parsippany, NJ 07054(US)

(72) Inventor: Thurman, Laurance Raymond
1207 Lake Street
Clute Texas 77531(US)
Inventor: Harris, James Burnia
12945 S. Post Oak No. 19
Houston Texas 77045(US)
Inventor: McAtee, Michael Richard
100 Lakeview No. 1125
Clute Texas 77531(US)

(74) Representative: Kinzel, Klaus, Dr. et al
BASF Aktiengesellschaft Patentabteilung
Carl-Bosch-Strasse 38
D-6700 Ludwigshafen(DE)

(54) **Coproduction of low molecular weight esters of alkanols.**

(57) A process for the production of low molecular weight alkanol esters is disclosed wherein an additional coproduct alkanol esters having a lower boiling point than the principal product is co-produced and utilized to remove water azeotropically.

EP 0 260 572 A1

Xerox Copy Centre

# COPRODUCTION OF LOW MOLECULAR WEIGHT ESTERS OF ALKANOLS

The subject invention relates to the production of organic esters. More particularly, the subject invention relates to coproduction of low molecular weight esters of alkanols.

In the past, low molecular weight esters of alkanols such as butyl acetate have been produced by a simple esterification reaction in the presence of a mineral acid catalyst. It is well known that under such conditions, an equilibrium is soon established wherein the ratio between alkanol and ester reaches a constant value, generally far on the side of the alkanol.

In order to shift the equilibrium in favor of the ester, the water produced as a product of the esterification reaction is removed as it is formed. For high boiling systems, i.e., those with alcohols and esters having boiling points substantially above that of water, the process becomes a simple matter of removing water by distillation. For low boiling systems, however, this approach is not feasible, and water in such systems is removed as an azeotrope with unreacted excess alcohol.

Removal of water by this means is economically unattractive for several reasons. First, the large excess of alcohol which must be utilized results in a relatively large throughput for only a small to modest output of product. Second, and most importantly, the alcohol/water azeotrope must itself be rectified in order that the relatively expensive alcohol component may be recycled. As many of the lower alkanols, i.e., n-propanol and n-butanol, particularly the former, are quite soluble in water, extensive and energy intensive distillations are required to separate the excess alcohol from this process stream.

According to the process of the present invention, low molecular weight alkanol esters may be advantageously produced without the necessity of utilizing excess alcohol as a means of water removal, through the coproduction of a low molecular weight alkanol ester with a still lower molecular weight ester. As a result of this process, not only is the system throughput per amount of product increased, but a coproduct ester is produced simultaneously.

According to the process of the subject invention, the production of a low molecular weight alkanol ester is facilitated through the coproduction of a lower molecular weight ester. Water produced in the esterification reaction is removed by means of azeotropic distillation wherein the lower molecular weight alkanol ester forms a major part of a binary, ternary, tertiary or higher azeotrope.

In the discussion which follows, by the term "low molecular weight alkanol ester" is meant those alkanol esters which have less than about 9 carbon atoms, preferably less than 7 carbon atoms, and particularly 6 or less carbon atoms in the ester molecule. These esters correspond to the formula:

$$R\text{-}O\text{-}\overset{\overset{O}{\|}}{C}\text{-}R^1$$

wherein R is a saturated or unsaturated organic radical selected from the group consisting of alkyl, alkenyl, cycloalkyl and cycloalkenyl radicals having from 1 to 6, preferably from 1 to 5, and particularly from 2 to 4 carbon atoms; wherein $R^1$ is a saturated or unsaturated organic radical selected from the group consisting of alkyl and alkenyl radicals having from 1 to 6 carbon atoms, preferably from 1 to 4 carbon atoms, and particularly from 1 to 3 carbon atoms, and wherein the total number of carbon atoms in R and $R^1$ is less than about 8.

Of the two or more alkanol esters which are coproduced in the process of the subject invention, those alkanol esters of lower molecular weight will be referred to hereinafter as "coproduct alkanol esters." Such coproduct alkanol esters have boiling points which are lower than the higher molecular weight alkanol ester just described which is the principal product. Such coproduct alkanol esters correspond to the formula:

$$R^2\text{-}O\text{-}\overset{\overset{O}{\|}}{C}\text{-}R^3$$

wherein $R^2$ is a saturated or unsaturated organic radical selected from the group consisting of alkyl, alkenyl, and cycloalkenyl radicals having from 1 to 5 carbon atoms, preferably from 1 to 4 carbon atoms, and in particular, from 2 to 3 carbon atoms; and wherein $R^3$ is a saturated or unsaturated organic radical selected from the group consisting of alkyl and alkenyl radicals having from 1 to 4 carbon atoms; wherein exclusively either R and $R^2$ or $R^1$ and $R^2$ are identical; and wherein the boiling point of the coproduct alkanol ester;

$$R^2\text{-}O\text{-}\overset{\overset{O}{\|}}{C}\text{-}R^3$$

is less than the alcohols R-OH and $R^2$-OH, the acids

$R^1-\overset{O}{\overset{\|}{C}}-OH$ and $R^3-\overset{O}{\overset{\|}{C}}-OH$,
and the principal alkanol ester product

$R-O-\overset{O}{\overset{\|}{C}}-R^1$.

Thus the alkanol esters of the subject invention are generally produced either from the esterification of a mixture of alkanols by a single organic acid; or by the esterification of a single alkanol by a mixture of acids. Although mixtures of both acids and alkanols might be used, the resulting product mixture would, in most cases, be exceedingly difficult to separate into the respective pure alkanol esters. Most preferably, byproduct alkanol streams, or mixtures of alkanols such as those derived from the oxo, Ziegler, or Fischer-Tropsch processes, are reacted with a single acid to form the alkanol esters of the subject invention.

More particularly, then, the subject invention relates to a process for the coproduction of alkanol esters under esterification conditions, in the presence of an esterification catalyst, comprising supplying to an esterification reactor an alkanol ester forming feed mixture which comprises either: a lower alkanol having the formula R-OH and two or more organic carboxylic acids having the formulas

$R^1-\overset{O}{\overset{\|}{C}}-OH$ and $R^3-\overset{O}{\overset{\|}{C}}-OH$;
<u>or</u> an organic carboxylic acid having the formula

$R^1-\overset{O}{\overset{\|}{C}}-OH$

and two or more lower alkanols having the formulas R-OH and $R^2$-OH; wherein R and $R^2$ are alkyl, alkenyl, alkynl, cycloalkyl, or cycloalkenyl radicals having from 1 to 6 carbon atoms and $R^2$ generally contains at least one carbon atom less than R; and wherein $R^1$ and $R^3$ are alkyl or alkenyl radicals having from 1 to 4 carbon atoms and $R^3$ contains at least one carbon atom less than $R^1$.

Water derived either from formation during esterification of the components of the alkanol ester forming feed or from water contained in the ester forming feed stream itself is removed by means of a binary, ternary, tertiary or higher azeotrope of water and at least 50 weight percent of an alkanol ester formed from the mixed acid feed, and having the formula

$R-O-\overset{O}{\overset{\|}{C}}-R^3$

or

$R^2-O-\overset{O}{\overset{\|}{C}}-R^1$.

From the azeotrope is separated a coproduct lower alkanol ester formed from the mixed acid feed and having the formula

$R-O-\overset{O}{\overset{\|}{C}}-R^3$

or

$R^2-O-\overset{O}{\overset{\|}{C}}-R^1$.

Finally, from the esterification reactor is separated a product alkanol ester formed from the feed and having the formula

$R-O-\overset{O}{\overset{\|}{C}}-R^1$.

Among the alkanols which are preferred for use in the subject invention, either alone or in mixtures, are saturated alkanols such as methanol, ethanol, 1-propanol, 2-propanol, 2-methylpropanol, 1-butanol, 2-butanol, 1-pentanol, 2-pentanol, 1-hexanol, 2-hexanol, cyclopentanol, and cyclohexanol; and unsaturated alkanols (actually alkenols and alkyneols) such as allyl alcohol, 1-butene-4-ol, 2-butene-1-ol, 3-cyclopentenol, 3-cyclohexenol, and propargyl alcohol.

Preferably used as mixtures of alkanols are 1-propanol and 1-butanol; 1-propanol and 2-methylpropanol; ethanol and 1-propanol; ethanol and 1-butanol; ethanol and 2-propanol; ethanol and 2-methylpropanol; 2-propanol and 1-butanol; 2-propanol and 2-methylpropanol; ethanol and 2-butanol; ethanol and 2-methylpropanol; ethanol and 1-pentanol; ethanol and 2-pentanol; 1-propanol and 1-pentanol; 1-propanol and 2-pentanol; 2-propanol and 1-pentanol; 2-propanol and 2-pentanol; ethanol, 1-propanol, and 1-butanol; ethanol, 1-propanol, and 2-methylpropanol; 1-propanol, 1-butanol and 1-pentanol; 1-propanol, 2-methylpropanol, and 1-butanol; and methanol, 1-propanol, 1-butanol and 1-pentanol.

The alkanols which are of use in the subject invention may be derived from natural sources, i.e., from the fermentation of natural sugars, starches, or other carbohydrates, but may also be obtained from one of the synthetic routes to alcohols, i.e., Fischer-Tropsch, Ziegler, or the so-called "oxo" process. In the latter process, for example, alkenes or mixtures of alkenes are reacted with carbon monoxide and hydrogen in the presence of suitable catalysts to form aldehydes which are then reduced to alcohols. When ethene and propene mixtures are used as the starting alkene, a product mix containing mostly 1-propanol and 1-butanol along with smaller amounts of methanol and higher alcohols may be formed. Such a product mixture is an ideal source of alkanols for the subject process.

Among the organic acids which may be used in the process of the subject invention, either alone or in mixtures, are organic alkanoic acids such as formic acid, acetic acid, propanoic acid and butyric acid; and alkenoic acids such as acrylic acid and methacrylic acid. Preferably used are acetic acid, butyric acid, acrylic acid and methacrylic acid.

Most preferably, the subject process is utilized to prepare the acetates of 1-propanol and 1-butanol which are derived from an alcohol by-product stream containing approximately equal quantities totalling about 50 to 60 percent by weight of 1-propanol and 1-butanol, the balance being mostly water. A typical by-product alkanol feed analysis may show the following composition, for example:

TABLE I

| Component | Weight % |
|---|---|
| 1-propanol | 25 |
| 1-butanol | 30 |
| water | 40 |
| other | 5 |
| | 100 |

Such a by-product alcohol stream may be used by itself as the coproduct alkanol ester feed, or it may be combined with a feed of relatively pure alkanol. In the case of the mixture in Table I, for example, a co-feed of n-butanol such that the total n-butanol content is about 70 weight percent or more is quite practical.

The alkanol feed may also be derived, as stated previously from oxo or other synthetic alkanol processes. The finished synthetic alkanol stream may be used in the case of processes producing solvent alkanols or, when higher, i.e., plasticizer or detergent range alkanols are produced, the alkanol by-products contained in the light fractions may be utilized. Such streams generally contain methanol, n-propanol, 2-propanol, n-butanol, and 2-methylpropanol (isobutanol). Such a stream may contain components as in Table II.

TABLE II

| Component | Weight % |
|---|---|
| methanol | 0.3 - 1.5 |
| n-propanol | 1 - 5 |
| 2-propanol | 0.3 - 2.0 |
| n-butanol | 60 - 70 |
| 2-methylpropanol | 10 - 20 |
| higher alcohols | 0.2 - 1.5 |

A notable feature of the subject invention is its ability to tolerate high levels of water in the alkanol feed. For example, when n-butanol and n-propanol are the primary alkanols, up to about 40 percent by weight water may be tolerated. In such a case, at levels higher than 40 percent, separation into organic and aqueous phases will likely occur in the feed itself.

In systems where water is more soluble, greater amounts may be tolerated, generally up to the maximum solubility of water in the alkanol mixture. In many cases, the upper limit will be fixed by limitations of existing process equipment, notably the distillation capacity of the esterification reactor column.

Referring to Figure 1, the mixed alkanol stream is fed to reactor R1 along with the esterifying acid which, for purposes of illustration, is acetic acid. Additional alkanol may be added, if desired, or if necessary to maintain system material balances. The reactor is maintained at a temperature suitable for esterification. In the case of 1-propanol and 1-butanol alkanol feed streams, a suitable temperature is from 90°C to about 130°C, preferably between 110°C and 126°C. The alkanol to acetic acid mole ratio is maintained at from about 1.02:1 to about 1.3:1, preferably from about 1.06:1 to about 1.1:1.

The esterification catalyst is generally sulfuric acid which is maintained at a concentration of from about 0.1 to about 2.0 percent by weight, preferably 0.4 to 0.6 percent by weight. The reactor is generally maintained at atmospheric pressure or below, for example from 400 to 760 torr, preferably from 600 to 760 torr. The product and azeotrope takeoffs are adjusted so as to produce a mean retention time of from 2 to 6, preferably from 3 to 5 hours.

Water contained in the feed as well as water produced during the esterification reaction is removed by azeotropic distillation in column C1 which is maintained at a head temperature of from about 82° to 90°C. The distillate consists mostly of water, 1-propanol, and 1-propyl acetate with the latter predominant. The distillate is collected in settling tank T1. The T1 organic phase, consisting mostly of 1-propyl acetate with small quantities of dissolved water and 1-propanol is washed with water in wash column W1 to free the 1-propyl acetate from 1-propanol to simplify production of pure 1-propyl acetate by distillation in Column C4.

The T1 aqueous layer is combined with the wash water from W1 and fed to column C3. The distillate from C3 consists of 1-propanol and 1-propyl acetate which is recycled to C1.

The reactor R1 effluent consists predominantly of 1-butyl acetate and 1-propyl acetate along with any higher acetates derived from higher alkanols in the alkanol feed. This reactor effluent is acidic in nature due to the acid esterification catalyst, and generally must be neutralized before subsequent treatment, particularly when other than glass lined equipment is utilized.

Following neutralization, where necessary, the crude esters along with the bottoms of column C4 are fractionated in column C5, with the lighter fraction, consisting mostly of 1-propyl acetate, being routed to column C4 for further purification. Product 1-butyl acetate is taken from C5 several theoretical plates above the bottoms which consists mainly of higher acetates such as amyl acetate.

Those skilled in the art of ester manufacture will recognize that many variations of the scheme illustrated by Figure 1 are possible. The operating pressures and temperatures of the various columns are dictated by the particular process stream being rectified. The azeotrope temperatures and composition for a wide variety of water/alkanol/alkanol ester systems are readily available from tables in standard chemical engineering texts.

A particularly advantageous modification to Figure 1 is the addition of a second reactor following reactor R1. The use of a second reactor allows higher overall throughput, especially when the alkanol feed contains considerable amounts of water. In this case, the greatest part of water is removed azeotropically from R1 along with some of the water produced by partial esterification. The R1 effluent then preferably contains about 0.5 percent or less by weight water.

As a result of this high level of water removal, esterification in the second reactor is greatly promoted. When such a two-stage process is utilized for the coproduction of butyl acetate and coproduct propyl acetate for example, the first reactor is generally maintained at a temperature of from 95° to 122°C, preferably from 110° to 120°C, while the second reactor is operated from 90° to 128°C, preferably from 116° to 126°C. When the two-reactor scheme is utilized, the residence time in each reactor is from 1 to 3 hours, preferably from 1.5 to 2.5 hours.

The composition of the final product and coproduct alkanol esters will depend upon the particular processing parameters and design chosen. Through the use of tandem reactors, for example, it is routinely possible to coproduce propyl acetate having very low color, as well as purity in excess of 98 weight percent, while producing large quantities of butyl acetate of the same quality as would be produced from a straight 1-butanol feed.

The following examples serve to illustrate the process of the subject invention.

Example 1

A 250 ml boiling flask fitted with a short fractionating column and a Dean-Stark trap is charged with 92.3 grams of butanol, 76.4 grams of acetic acid, 7.7 grams of propanol, and 0.88 grams of sulfuric acid. The reaction mixture is refluxed at 80°C to remove the reaction (and dissolved) water in the boiling flask. After completion of the esterification reaction, the organics in the Dean-Stark trap have a propyl acetate content of 70 to 80 weight percent. The water content in the boiling flask is less than 1 percent.

Example 2

A 500 ml boiling flask fitted with a short fractionating column and a Dean-Stark trap and having a bottom takeoff is utilized as the first esterification vessel. The bottoms from the first esterification vessel are metered to a second, identical 500 ml boiling flask which serves as a second esterification vessel. The feed rate to the second vessel is adjusted to the same rate as the feed to the first vessel. A fractionating column is attached to the second vessel.

The alkanol feed to the first vessel consists of 35 weight percent 1-butanol, 30 weight percent 1-propanol, and 35 percent water. The organic carboxylic acid feed to the first vessel is acetic acid and is adjusted to maintain an alkanol to acetic acid mole ratio of approximately 1.07. The alkanol and acetic acid feed rate is adjusted to achieve a first vessel residence time of 1.5 hours at a reaction vessel temperature of about 115°C. The fractionating head temperature is 85°C.

The second reactor vessel is preferably maintained at about 120°C. The takeoff from the second reactor vessel, following neutralization, is fed to a fractionating column filled with beryl saddles. The takeoff from the lower section of the column consists of 1-butyl acetate with a purity in excess of 99 percent.

The organics in the Dean-Stark trap are extracted with water in a separatory funnel to remove any unreacted alkanol. The organic layer is combined with the distillate from the fractionating column following the second reactor vessel and distilled. The distillate, after lights removal, consists of 1-propyl acetate in c.a. 95 percent yield and a purity of >98 weight percent.

The water layer from the Dean-Stark trap and the water from extraction of the organic layer contain a small quantity of 1-propyl acetate and a larger quantity of 1-propanol. Analysis of the water and organics layers in the Dean-Stark trap indicate a composition containing, in weight percentage, approximately 20 percent water, 20 percent 1-propanol, 58 percent 1-propyl acetate, and about 2 percent n-butyl acetate. This composition corresponds closely with the 82°C ternary azeotrope of water, 1-propanol, and 1-propyl acetate which has a weight composition of $H_2O$/1-propanol/1-propyl acetate of 21/20/60.

## Claims

1. A process for the coproduction of alkanol esters under esterification conditions comprising:

a) supplying to an esterification reactor a composition selected from the group consisting of

i) an alkanol ester forming feed mixture comprising a lower alkanol having the formula R-OH and two or more organic carboxylic acids having the formulas

$R^1-\overset{O}{\overset{\|}{C}}-OH$ and $R^3-\overset{O}{\overset{\|}{C}}-OH$;

and

ii) an alkanol ester forming feed mixture comprising an organic carboxylic acid having the formula

$R^1-\overset{O}{\overset{\|}{C}}-OH$

and two or more lower alkanols having the formulas R-OH and $R^2$-OH;

wherein R and $R^2$ are alkyl, alkenyl, alkynl, cycloalkyl, or cycloalkenyl radicals having from 1 to 6 carbon atoms and $R^2$ contains at least one carbon atom less than R; and wherein $R^1$ and $R^3$ are alkyl or alkenyl radicals having from 1 to 4 carbon atoms and $R^3$ contains at least one carbon atom less than $R^1$;

b) removing water derived from

iii) formation during esterification of the components of said alkanol ester forming feed; and

iv) water contained in said ester forming feed stream

by means of a binary, ternary, tertiary or higher azeotrope of water and at least 50 weight percent of an alkanol ester formed from said feed i) and having the formula

$R-O-\overset{O}{\overset{\|}{C}}-R^3$

or from said feed ii) and having the formula

$R^2-O-\overset{O}{\overset{\|}{C}}-R^1$;

c) separating from said azeotrope a coproduct lower alkanol ester formed from feed i) and having the formula

$R-O-\overset{O}{\overset{\|}{C}}-R^3$

or from feed ii) and having the formula

$R^2-O-\overset{O}{\overset{\|}{C}}-R^1$; and

d) separating from said esterification reactor a product alkanol ester formed from said feed and having the formula

$R-O-\overset{O}{\overset{\|}{C}}-R^1$.

2. The process of claim 1 wherein the alkanols R-OH and $R^2$-OH of step a)ii) are selected from the group consisting of methanol, ethanol, 1-propanol, 2-propanol, 1-propene-3-ol, 2-methylpropanol, 1-butanol, 2-butanol, 1-pentanol, 2-pentanol and cyclohexanol.

3. The process of claim 1 wherein R-OH is 1-butanol and $R^2$-OH is selected from the group consisting of methanol, ethanol, 1-propanol, 2-propanol, and mixtures thereof.

4. The process of claim 1 wherein said lower alkanols R-OH and $R^1$-OH are respectively selected from the group consisting of 1-butanol and 1-propanol; and 2-methylpropanol and 1-propanol.

5. The process of claim 1 wherein said azeotrope is at least a ternary azeotrope containing as its third components an unreacted lower alkanol R-OH derived from feed i) or an unreacted lower alkanol $R^2$-OH derived from feed ii).

6. The process of claim 1 wherein said organic carboxylic acids

$R^1-\overset{\overset{O}{\|}}{C}-OH$ and $R^3-\overset{\overset{O}{\|}}{C}-OH$

are selected from the group consisting of formic acid, acetic acid, acrylic acid, propanoic acid, and butyric acid.

7. The process of claim 1 wherein said organic carboxylic acid of step a)ii) is selected from the group consisting of acetic acid and acrylic acid.

8. The process of claim 1 wherein said lower alkanols R-OH and $R^2$-OH of step a)ii) are 1-butanol and 1-propanol, respectively, and wherein said organic carboxylic acid of step a)ii) is acetic acid.

9. The process of claim 1, further comprising supplying the output of said esterification reactor after a residence time of from 1 to 3 hours to a second esterification reactor.

10. The process of claim 9 wherein the input to said second esterification reactor contains less than about 1.0 percent by weight of water.

11. The process of claim 10 wherein said input to said second reactor contains less than about 0.7 percent by weight water.

12. The process of claim 9 wherein the residence times for both the esterification reactor and the second esterification reactor is from 1.5 to 2.5 hours.

13. The process of claim 9 wherein said lower alkanols R-OH and $R^2$-OH of step a)ii) are 1-butanol and 1-propanol.

14. The process of claim 9 wherein said organic carboxylic acid is selected from the group consisting of formic acid, acetic acid, acrylic acid, propanoic acid, and butyric acid.

15. The process of claim 13 wherein said organic carboxylic acid is selected from the group consisting of formic acid, acetic acid, acrylic acid, propanoic acid, and butyric acid.

16. The process of claim 13 wherein said organic carboxylic acid is acetic acid.

17. The process of claim 1 wherein said alkanol ester formed from alkanol R-OH and organic carboxylic acid

$R^1-\overset{\overset{O}{\|}}{C}-OH$

and having the formula

$R-O-\overset{\overset{O}{\|}}{C}-R^1$

has a boiling point at 760 torr of less than about 160°C.

18. The process of claim 9 wherein said alkanol ester formed from alkanol R-OH and organic caboxylic acid

$R^1-\overset{\overset{O}{\|}}{C}-OH$

and having the formula

$R-O-\overset{\overset{O}{\|}}{C}-R^1$

has a boiling point at 760 torr of less than about 160°C.

19. A process for the coproduction of alkanol esters under esterification conditions, comprising

a) supplying to an esterification reactor an alkanol ester forming feed mixture comprising i) an organic carboxylic acid

$R^3-\overset{\overset{O}{\|}}{C}-OH$, and

ii) at least two alkanols R-OH and $R^2$-OH;

b) removing water by means of a binary, ternary, tertiary or higher azeotrope of water and an alkanol ester having the formula

$R^2-O-\overset{\overset{O}{\|}}{C}-R^3$;

c) separating from said azeotrope said alkanol ester having the formula

$R^2-O-\overset{\overset{O}{\|}}{C}-R^3$; and

d) separating from said esterification reactor a product alkanol ester formed from said feed and having the formula

$$R\text{-}O\text{-}\overset{O}{\overset{\|}{C}}\text{-}R^3,$$

wherein said alkanol ester

$$R^2\text{-}O\text{-}\overset{O}{\overset{\|}{C}}\text{-}R^3$$

has a lower boiling point than said alkanol ester

$$R\text{-}O\text{-}\overset{O}{\overset{\|}{C}}\text{-}R^3,$$

and wherein said alkanol esters

$$R\text{-}O\text{-}\overset{O}{\overset{\|}{C}}\text{-}R^3 \text{ and } R^2\text{-}O\text{-}\overset{O}{\overset{\|}{C}}\text{-}R^3$$

each contain less than about 9 carbon atoms.

20. The process of claim 19 wherein R-OH and $R^2$-OH are n-butanol and 2-methylpropanol, respectively.

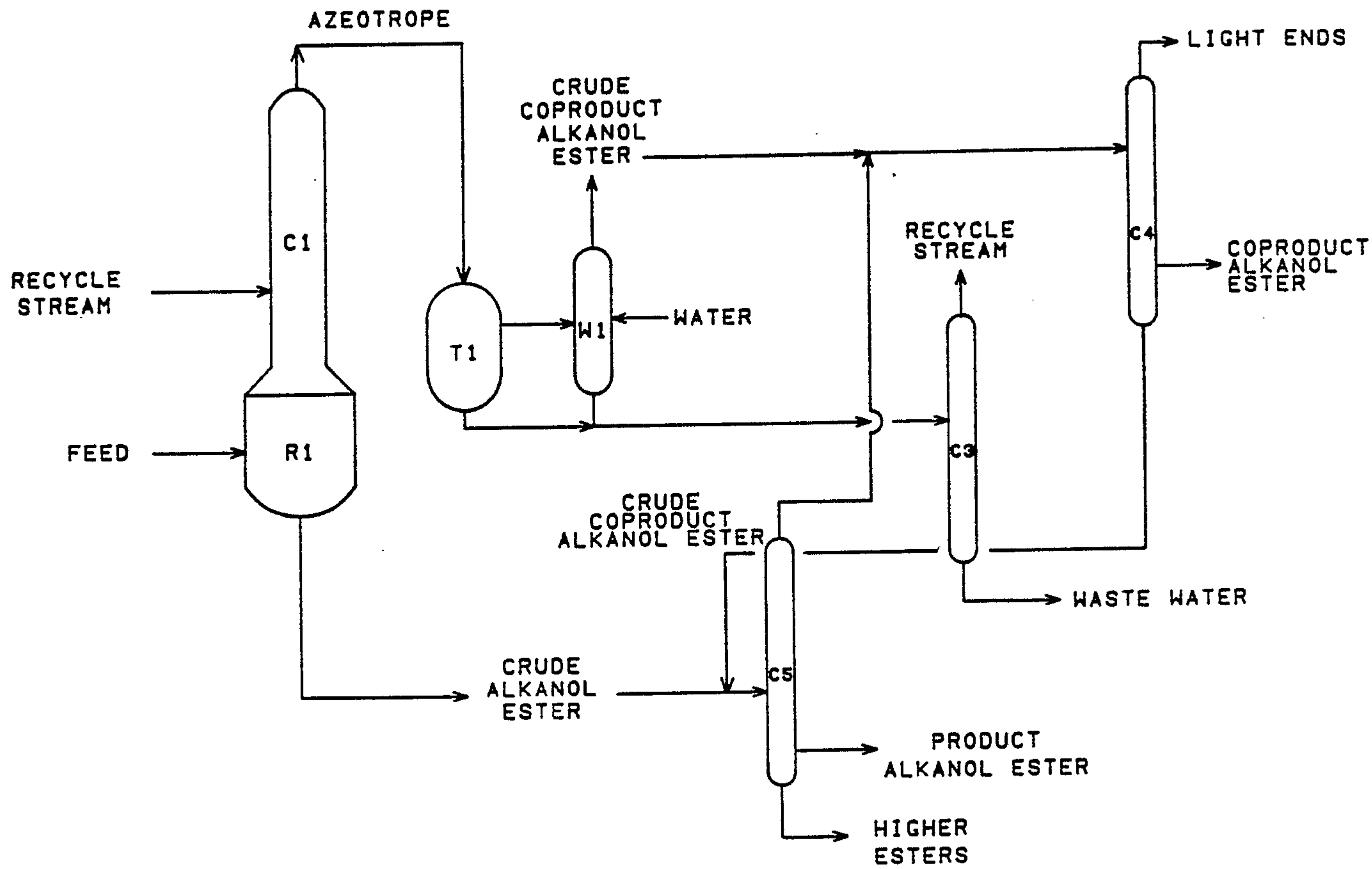
AZEOTROPE
LIGHT ENDS
CRUDE COPRODUCT ALKANOL ESTER
C1
RECYCLE STREAM
C4
RECYCLE STREAM
COPRODUCT ALKANOL ESTER
T1
W1
WATER
FEED
R1
C3
CRUDE COPRODUCT ALKANOL ESTER
WASTE WATER
CRUDE ALKANOL ESTER
C5
PRODUCT ALKANOL ESTER
HIGHER ESTERS

FIGURE ONE

European Patent Office

# EUROPEAN SEARCH REPORT

Application number

EP 87113080.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | GB - A - 1 438 410 (BP CHEMICALS)<br>* Totality; especially claims 1-5 and comparative test 1 *<br>---- | 1 | C 07 C 69/14<br>C 07 C 67/08 |
| | | | **TECHNICAL FIELDS SEARCHED (Int. Cl.4)** |
| | | | C 07 C 69/00 |

The present search report has been drawn up for all claims

| Place of search | Date of completion of the search | Examiner |
|---|---|---|
| VIENNA | 30-11-1987 | HOFBAUER |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82